## Europäisches Patentamt
## European Patent Office
## Office européen des brevets

(19)

(11) Veröffentlichungsnummer: **0 173 800**
**B1**

(12) **EUROPÄISCHE PATENTSCHRIFT**

(45) Veröffentlichungstag der Patentschrift:
23.08.89

(51) Int. Cl.⁴: **G 01 N 27/46**

(21) Anmeldenummer: **85106572.2**

(22) Anmeldetag: **29.05.85**

(54) Elektrochemische Messzelle mit Zusatzelektrode.

(30) Priorität: 07.09.84 DE 3432950

(43) Veröffentlichungstag der Anmeldung:
12.03.86 Patentblatt 86/11

(45) Bekanntmachung des Hinweises auf die Patenterteilung:
23.08.89 Patentblatt 89/34

(84) Benannte Vertragsstaaten:
DE FR GB NL

(56) Entgegenhaltungen:
EP-A-0 077 054
DE-A-2 101 339
DE-A-2 539 771
DE-A-2 930 663
GB-A-979 117

(73) Patentinhaber: **Drägerwerk Aktiengesellschaft, Moislinger Allee 53- 55, D-2400 Lübeck 1 (DE)**

(72) Erfinder: **Hölscher, Uvo, Dr., Stettiner Strasse 6a, D-2406 Stockelsdorf (DE)**

EP 0 173 800 B1

## Beschreibung

Die Erfindung betrifft eine elektrochemische Meßzelle, enthaltend eine Gegenelektrode und eine Meßelektrode in einem Elektrolyten, welcher durch eine Diffusionsmembran von dem Außenraum getrennt ist, und mit einer Vorrichtung zur zusätzlichen Ableitung eines äußeren elektrischen Potentials.

Eine elektrochemische Meßzelle der genannten Art ist aus der EP-A2-0 077 054 bekannt. Bei dieser Meßzelle ist eine erste Elektrodenanordnung, welche durch eine Diffusionsmembran gegenüber dem Außenraum abgeschlossen ist, von einer zweiten Elektrode ringförmig umgeben, wobei die dazugehörige Gegenelektrode auf der Rückseite des Meßzellenkopfes angeordnet ist. Die zusätzliche Elektrodenanordnung kann beispielsweise dazu eingesetzt werden, eine bioelektrische Größe (EKG) aufzunehmen.

Bei dem bekannten Meßwertaufnehmer muß die zusätzliche ringförmige Elektrode in einem den Meßkopf umfassenden Gewindering aufgenommen werden. Dies hat zur Folge, daß die Meßzelle eine solche Größe aufweist, daß sie ungeeignet ist, in kleinere Öffnungen eingeführt werden zu können. Darüberhinaus wird bei sonst gleicher Gesamtgröße die Klebefläche zur Befestigung der Meßzelle auf eine Unterlage, wie zum Beispiel menschliche oder tierische Haut, derart verringert, daß ein zuverlässiges Haften unter häufig in der Praxis auftretenden erschwerten Bedingungen nicht mehr gewährleistet ist.

Der Erfindung liegt die Aufgabe zugrunde, die bekannten Meßwertaufnehmer derart zu verbessern, daß eine Vorrichtung zur zusätzlichen Ableitung eines äußeren elektrischen Potentials ohne eigenen Platzbedarf an der Meßzelle angeordnet werden kann, wodurch die zur Verfügung stehende Klebefläche vergrößert wird.

Die Lösung der Aufgabe wird dadurch erzielt, daß die Vorrichtung auf der dem Außenraum zugewandten Fläche der Diffusionsmembran angeordnet ist.

Der weiteren vorteilhaften Ausgestaltung der Erfindung dienen die in den abhängigen Ansprüchen gekennzeichneten Merkmale.

Mit der erfindungsgemäßen Anordnung der Vorrichtung erhält man eine großflächige Elektrode zur Ableitung eines äußeren elektrischen Potentials, welche durch ihre Anbringung auf der Außenfläche der Diffusionsmembran eine für die Anwendung geschickte Flächenausnutzung darstellt.

In vorteilhafter Weise kann die Vorrichtung aus einem Belag bestehen, welcher auf die Außenfläche der Membran aufgebracht ist, wie z. B. aus einem Metall- oder Graphitbelag, wobei auch beispielsweise ein Drahtgeflecht oder Gitternetz aus leitfähigem Material eingesetzt werden kann.

In weiterer vorteilhafter Ausgestaltung der Erfindung kann die Diffusionsmembran selbst aus leitfähigem Kunststoff ausgebildet sein. Ein solcher leitfähiger Kunststoff ist vorzugsweise Polypyrrol. Es sind aber auch andere bekannte leitfähige Kunststoffe einsetzbar. Enthalten solche leitfähigen Kunststoffe darüberhinaus elektrochemisch aktive Substanzen oder sind sie selber elektrochemisch aktiv, könnte eine solche Diffusionsmembran auch gleichzeitig als Meßelektrode der elektrochemischen Zelle dienen.

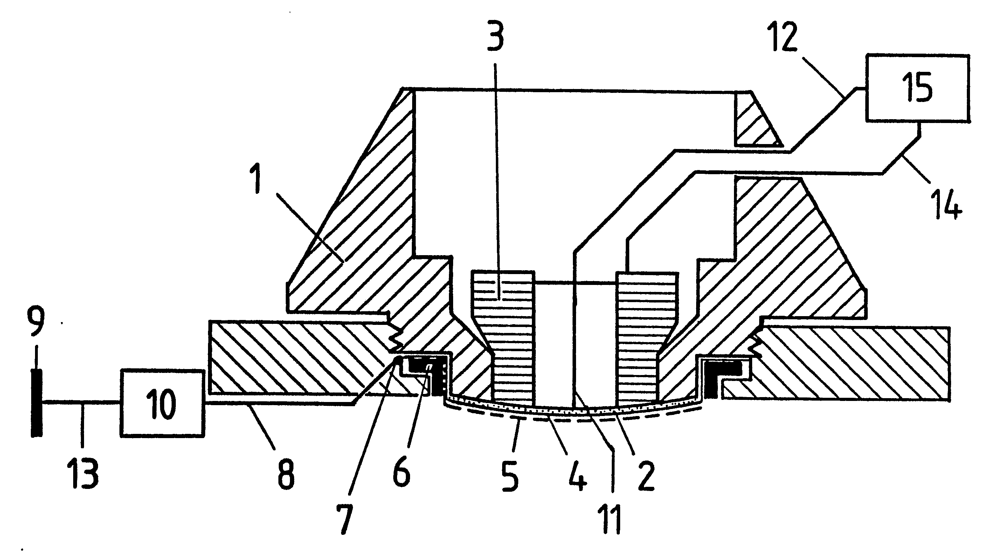

Ein Ausführungsbeispiel der Erfindung ist in der Zeichnung dargestellt und wird im folgenden beschrieben.

In der im Schnitt dargestellten elektrochemischen Meßzelle ist ein Gehäuse 1 dargestellt, welches den Elektrolyten 2 und die Gegenelektrode 3 enthält und das gegenüber dem Außenraum mit einer Diffusionsmembran 4 abgeschlossen ist. Die Außenfläche der Diffusionsmembran 4 ist mit einem elektrisch leitfähigen Belag 5 überzogen, und zwar so weit, daß der Spannring 6 in elektrischem Kontakt mit der beschichteten Diffusionsmembran 4 ist, so daß an ihm über den Kontakt 7 und die Zuleitung 8 das äußere elektrische Potential abgeleitet und einer Auswerteeinheit 10 zugeleitet wird, deren zweiter Meßeingang über die Verbindungsleitung 13 an eine Bezugselektroden angeschlossen ist. Die Meßelektrode 11 wird über die Zuleitung 12, und die Gegenelektrode 3 über die Zuleitung 14 mit dem Meßgerät 15 zur Auswertung des Meßsignals der elektrochemischen Zelle verbunden.

## Patentansprüche

1. Elektrochemische Meßzelle, enthaltend eine Gegenelektrode (3) und eine Meßelektrode (11) in einem Elektrolyten (2), welcher durch eine Diffusionsmembran (4) von dem Außenraum getrennt ist, und eine Vorrichtung (5) zur zusätzlichen Ableitung eines äußeren elektrischen Potentials, dadurch gekennzeichnet, daß die Vorrichtung auf der dem Außenraum zugewandten Fläche der Diffusionsmembran (4) angeordnet ist.

2. Elektrochemische Meßzelle nach Anspruch 1, dadurch gekennzeichnet, daß die Vorrichtung aus einem elektrisch leitfähigen, gasdurchlässigen Belag (5) auf der Diffusionsmembran (4) gebildet ist.

3. Elektrochemische Meßzelle nach Anspruch 1, dadurch gekennzeichnet, daß die Vorrichtung eine aus elektrisch leitfähigem Kunststoff bestehende Diffusionsmembran (4) ist.

4. Elektrochemische Meßzelle nach Anspruch 3, dadurch gekennzeichnet, daß die Diffusionsmembran (4) aus Polypyrrol besteht.

## Revendications

1. Cellule de mesure électrochimique, contenant une contre-électrode (3) et une électrode de mesure (11) dans un électrolyte (2), qui est séparé de l'espace extérieur par une membrane de diffusion (4), et un dispositif (15) pour la dérivation complémentaire d'un potentiel électrique extérieur, caractérisée en ce que le dispositif est disposé sur la face de la membrane de diffusion (4) qui est tournée vers l'espace extérieur.

2. Cellule de mesure électrochimique selon la revendication 1, caractérisée en ce que le dispositif est réalisé sous la forme d'une garniture (5) électriquement conductrice et perméable aux gaz, appliquée sur la membrane de diffusion (4).

3. Cellule de mesure électrochimique selon la revendication 1, caractérisée en ce que le dispositif est une membrane de diffusion (4) réalisée en matière plastique électriquement conductrice.

4. Cellule de mesure électrochimique selon la revendication 3, caractérisée en ce que la membrane de diffusion (4) est réalisée en polypyrrol.

## Claims

1. An electrochemical measuring cell, comprising a counter electrode (3) and a measuring electrode (11) in an electrolyte (2), which is separated from the atmosphere by a diffusion membrane (4), and a device (5) for additionally drawing off an external electric potential, characterised in that the device is arranged on the surface of the diffusion membrane (4) facing the outside.

2. An electrochemical measuring cell according to claim 1, characterised in that the device is formed from an electrically conductive, gas-premeable coating (5) on the diffusion membrane (4).

3. An electrochemical measuring cell according to claim 1, characterised in that the device is a diffusion membrane (4) made of electrically conductive plastics material.

4. An electrochemical measuring cell according to claim 3, characterised in that the diffusion membrane (4) is made of polypyrrole.